# FASCICULE DE BREVET EUROPEEN

(11) **EP 3 247 206 B1**
(45) Date de publication et mention de la délivrance du brevet: **14.11.2018**
(21) Numéro de dépôt: 15823719.8
(22) Date de dépôt: 24.12.2015
(51) Int. Cl.: A01L 11/00

(54) **PROCEDE DE FABRICATION D'UN FER A CHEVAL**
VERFAHREN ZUR HERSTELLUNG EINES HUFBESCHLAGES
METHOD OF MAKING A HORSESHOE

(30) Priorité: 20.01.2015 FR 1550442
(43) Date de publication de la demande: 29.11.2017
(73) Titulaire: Value Feet, 33610 Canejan (FR)
(72) Inventeur: ROORYCK, Thibaut, 33610 Canejan (FR); ROORYCK, Maxime, 33610 Canejan (FR)
(74) Mandataire: Cabinet Plasseraud
(86) Numéro de dépôt international: PCT/FR2015/053753
(87) Numéro de publication internationale: WO 2016/116677

(56) Documents cités:
- EP-A1- 0 545 961
- WO-A1-2010/086190

## Description

### Domaine de l'invention

La présente invention concerne un procédé de fabrication d'un fer à cheval.

La présente invention concerne encore un système d'assistance à la réalisation d'un fer à cheval, pour la mise en oeuvre de ce procédé de fabrication.

### Arrière-plan technologique

Afin de préserver les qualités du pied du cheval, il est connu de protéger le pied en posant sur le sabot un fer à cheval.

Si autrefois, le façonnage d'un fer à cheval relevait exclusivement de la compétence du maréchal-ferrant, les fers ordinaires sont de plus en plus fabriqués en usine.

Bien que les fers à cheval ainsi obtenus présentent une forme sensiblement uniforme et plane, ces fers assurent un gain de temps non négligeable au maréchal-ferrant.

Afin d'alléger les fers à cheval tout en assurant une tenue adéquate, les fers fabriqués en usine sont, pour la compétition, typiquement réalisés en aluminium, et non plus en acier.

Toutefois, de tels fers étant alors difficilement façonnables, le maréchal-ferrant doit typiquement choisir un fer dont la forme et les dimensions sont les plus proches du fer à cheval adapté au sabot à ferrer.

Par ailleurs, de tels fers ne peuvent prendre en compte des imperfections ou pathologies du sabot.

Ainsi, dès qu'il est nécessaire de produire une ferrure orthopédique, celle-ci est formée par le maréchal-ferrant et est, en conséquence, notamment réalisée en acier.

Même lorsque ce façonnage peut être obtenu par reprise d'un fer préformé, cette opération est particulièrement longue et pénible pour le maréchal-ferrant, et de surcroît, onéreuse.

En outre, une ferrure orthopédique peut être adaptée pour corriger un ou plusieurs problèmes principaux sans traiter des problèmes secondaires engendrés par ces problèmes principaux. (Voir par exemple EP 0 545 961 B1.)

La vérification d'une zone douloureuse du pied du cheval par palpation entraîne également une réaction à la douleur du cheval. Comme il peut être nécessaire de revenir à plusieurs reprises sur le pied douloureux pour confirmer le diagnostic, cette vérification est spécialement inconfortable pour le cheval. Elle est en outre peu précise.

On constate aussi qu'il peut s'avérer difficile de détecter un problème de santé sur une jambe ou le pied d'un cheval, par exemple parce qu'il n'existe aucun signe clinique apparent (inflammation infraclinique) ou parce que l'infection précède l'apparition de signes de boiterie, par exemple.

Il convient toutefois de soulager le cheval pour éviter que son état de santé ne s'aggrave.

Il existe donc un besoin pressant pour un procédé permettant de fabriquer un fer à cheval dont la structure et/ou la forme sont particulièrement adaptées au pied d'un cheval à ferrer tout en prenant en compte son état physiologique.

### Objet de l'invention

La présente invention vise à pallier les inconvénients de l'art antérieur et à répondre aux contraintes ci-dessus énoncées en proposant un procédé de fabrication d'un fer à cheval, simple dans sa conception et dans son mode opératoire, fiable et économique, permettant de déterminer les paramètres d'un fer à cheval les plus adaptés à un pied donné d'un cheval tout en assurant une prise en compte non seulement de la morphologie mais également des pathologies éventuelles de ce pied de manière à garantir un confort optimal pour le cheval.

Un autre objet de la présente invention est un tel procédé qui soit non invasif et rapide, et donc non contraignant pour le cheval.

Encore un objet de la présente invention est un système d'assistance à la réalisation d'un fer à cheval, pour la mise en oeuvre de ce procédé de fabrication d'un fer à cheval.

### BREVE DESCRIPTION DE L'INVENTION

A cet effet, l'invention concerne un procédé dans lequel on réalise les étapes suivantes :
a) visualiser au moins en partie le sabot à ferrer pour déterminer des mesures nécessaires à la réalisation d'un fer adapté à ce sabot,
b) traiter lesdites mesures pour en déduire une forme prévisionnelle du fer à cheval,
c) enregistrer des paramètres définitifs dudit fer à cheval, et
d) réalisation du fer à cheval à partir desdits paramètres définitifs.
Selon l'invention,
- avant l'étape b), on détermine au moyen d'un détecteur infrarouge l'existence éventuelle d'au moins une zone d'inflammation et/ou d'au moins une zone de réduction de la circulation sanguine du pied du cheval, dont le sabot est destiné à recevoir ledit fer, et
- on tient compte à l'étape b), de l'existence d'au moins une telle zone pour déterminer ladite forme prévisionnelle et/ou ladite structure dudit fer à cheval.

De manière connue, et préalablement à l'étape a), on réalise, de préférence, une préparation du cheval comportant une étape de nettoyage du sabot, de coupe de la corne, ...

De manière avantageuse, on utilise un détecteur infrarouge thermique pour déterminer l'existence éventuelle d'au moins une zone d'inflammation et/ou d'au moins une zone de réduction de la circulation sanguine du pied du cheval.
Une "zone chaude" indique une inflammation ou une circulation du sang accrue. Ces zones chaudes sont typiquement observées dans la peau au-dessus d'une blessure. Une "zone froide" correspond quant à elle à une réduction de l'alimentation sanguine généralement due à un gonflement ou à la présence de tissus cicatriciels.
La détection de ces zones de manière non invasive par thermographie infrarouge permet avantageusement de diagnostiquer de manière précoce une éventuelle pathologie de manière à adapter la structure et/ou la forme du fer à cheval pour soulager le pied du cheval et ne pas aggraver sa condition.

Dans différents modes de réalisation particuliers de ce procédé, chacun ayant ses avantages particuliers et susceptibles de nombreuses combinaisons techniques possibles:
- l'étape de détection d'au moins une éventuelle zone d'inflammation et/ou d'au moins une zone de réduction de la circulation sanguine du pied du cheval est réalisée simultanément à la visualisation du sabot à ferrer.
On acquiert ainsi une image thermographique du dessous du pied à ferrer conjointement à sa visualisation.
Ces étapes de mesure et de détection étant réalisées simultanément, on réduit ainsi drastiquement le temps nécessaire à l'élaboration de la forme et/ou de la structure du fer à cheval en travaillant à partir de deux images fournissant des informations complémentaires.
De manière avantageuse, ces images visuelle et thermique étant obtenues avec une même ligne de visée du dessous du pied du cheval, on pourra superposer les informations issues de ces deux images pour déterminer la forme prévisionnelle optimale du fer à cheval.
- à l'étape a), on acquiert une image numérique d'au moins une partie du sabot à ferrer,
- on réalise également avant l'étape b) au moins une image radiographique dudit pied du cheval au moyen d'une source de rayons X portable et d'un dispositif d'imagerie par rayons X pour déterminer une zone éventuelle d'affection.
Bien entendu, lorsque la ou les images radiographiques révèlent l'existence d'une telle zone d'affection, on tient compte à l'étape b) de cette zone pour déterminer ladite forme prévisionnelle et/ou ladite structure dudit fer à cheval.
Une telle zone d'affection peut être liée à une affection articulaire, lésion, fracture,....
- avant l'étape c), on réalise les étapes suivantes : réaliser un patron à partir des paramètres dudit fer à cheval obtenus à l'étape b), placer ce patron sur le sabot à ferrer et déterminer les paramètres définitifs dudit fer à cheval à fabriquer.
A titre illustratif, ce patron est obtenu au moyen d'une découpeuse actionnée sous commande numérique ou d'une imprimante telle qu'une imprimante numérique. S'agissant par exemple d'une simple impression de la forme prévisionnelle du fer à cheval sur une feuille, on découpe la feuille imprimée pour obtenir le patron et le positionner ce dernier sur le sabot pour s'assurer de sa qualité ou pour ajuster ses paramètres.
Ce patron peut encore être obtenu au moyen d'un système pour la fabrication d'objets tridimensionnels, et notamment une imprimante tridimensionnelle, telle qu'une imprimante thermique de fabrication d'un modèle tridimensionnel par dépôt séquentiel d'une pluralité de couches de section transversale.
On s'assure ainsi, en direct, grâce à la réalisation de ce patron en présence du cheval, de la détermination du bon jeu de paramètres et de la qualité finale du fer à cheval qui sera produit à partir du jeu de paramètres définitifs.
- l'étape b) comporte une étape de correction des paramètres du fer à cheval correspondant au moins à un ajustement de la courbe externe, ou encore du contour, du fer à cheval.
On tient compte ainsi des particularités de fabrication du fer à cheval déterminées par le maréchal-ferrant. A titre purement illustratif, il peut s'agir de la prise en compte de la déformation du sabot lors de sa prise d'appui sur le sol.
- le pied du cheval présentant au moins une zone douloureuse ou une zone de réduction de la circulation sanguine, à l'étape b) on allège la structure du fer au droit de ladite au moins une zone de sorte à soulager celle-ci,
- à l'étape d), on réalise au moins une partie du fer à cheval par un procédé de fabrication par impression tridimensionnelle.
A titre purement illustratif, le fer à cheval ayant une structure multicouche, au moins une de ces couches est obtenue par un procédé de fabrication par impression tridimensionnelle puis la couche ainsi obtenue est assemblée à au moins deux autres couches, par exemple par collage, pour former le fer à cheval.
La couche ainsi obtenue peut être métallique (Aluminium, fer, titane, ...) ou en polymère.
Par exemple, elle peut être obtenue à partir d'une poudre métallique agglomérable, encore appelée « poudre frittable ».
- on marque la surface externe dudit fer avec un identifiant unique dudit fer à cheval et éventuellement un identifiant du pied du cheval à ferrer auquel est destiné ledit fer à cheval.
Cet identifiant unique comporte un signe obtenu par réaction du matériau constitutif de ladite face externe du fer à cheval à une substance chimique ou à l'application d'une source de chaleur telle qu'un faisceau laser.
Ce signe peut présenter un relief de surface tel qu'au moins un creux.
De préférence, ce signe comprend un trait vertical, un trait oblique, un ou plusieurs cercles, un ou plusieurs chiffres, une ou plusieurs lettres, un code barre, un symbole et des combinaisons de ces éléments.
- on réalise une finition de la couleur du fer à cheval ainsi obtenu par traitement d'au moins une partie de la surface externe dudit fer, ledit traitement comportant une étape d'anodisation et une étape de coloration, ou par dépose sur au moins une partie de la surface externe dudit fer d'un revêtement décoratif présentant une couleur déterminée.
A titre purement illustratif, la coloration de cette surface peut être effectuée par un procédé de coloration par adsorption, un procédé de coloration électrolytique, un procédé de coloration par interférence ou une combinaison quelconque de ces procédés.
Alternativement, après avoir anodisé la surface externe du fer à cheval, on pourra exposer cette pièce à un procédé de colmatage à froid ou à chaud tel qu'en immergeant cette pièce dans de l'eau désionisée, à une température de l'ordre de 90°C à 100°C, un colorant tel que du bleu de méthylène par exemple qui conférera une couleur bleue au fer à cheval.
A titre d'exemple, le procédé de coloration électrolytique comprend le dépôt électrolytique des particules d'une solution de sel métallique au niveau des pores de couche d'oxyde de la surface du fer.
- déposant un revêtement décoratif résistant à l'usure, on choisit un métal précieux parmi le groupe comprenant l'Or, l'Argent, le Platine, le Palladium, le Rhodium, l'Iridium, l'Osmium, le Rhénium, le Ruthénium et/ou d'un alliage d'un de ces métaux avec un ou plusieurs autres métaux.

A titre purement illustratif, on dépose galvaniquement une couche de base constituée d'un alliage de métal précieux.
De plus, pour renforcer la résistance d'un placage d'or par exemple, on peut déposer galvaniquement une couche superficielle d'alliage d'or ayant une pureté supérieure ou égale telle que 22 carats.
Alternativement, pour réaliser ce revêtement décoratif couleur de l'or qui soit résistant à l'usure, on dépose sous vide, lors d'une première étape, sur la surface du fer à cheval, au moins une première couche de nitrure de titane, puis au cours d'une seconde étape, on active cette première couche par bombardement ionique sous vide de sorte qu'elle soit apte à recevoir, par la suite une couche d'or ou d'alliage d'or de pureté élevée, déposée par procédé galvanique, ayant la couleur définitive souhaitée. Lors de cette seconde étape, on dépose, au moins en partie simultanément une seconde couche fine d'or et/ou d'un alliage d'or. Ce dépôt d'atomes d'or s'effectue sous vide par évaporation, par projection ionique ou par pulvérisation cathodique, tout en continuant à effectuer un bombardement ionique de la surface de nitrure de titane. Au cours de cette seconde étape, on réduit progressivement la puissance de ce bombardement ionique.
L'alliage d'or est, de préférence, à carats élevés, par exemple un alliage d'or à au moins 22 carats comportant, comme élément d'alliage, de l'Indium, du Nickel, du Cobalt, du Cadmium, du Cuivre, de l'Argent, du Palladium, du Zinc ou de l'Antimoine.

La présente invention concerne encore un système d'assistance à la réalisation d'un fer à cheval, pour la mise en oeuvre du procédé tel que décrit précédemment.
Selon l'invention, ce système comprend :
- un dispositif optique,
- un détecteur infrarouge,
- ledit système étant agencé de sorte qu'au moins ledit dispositif optique visualise la partie dudit sabot destinée à recevoir ledit fer à cheval, et
- des moyens assurant le transfert des données acquises par ledit détecteur infrarouge et ledit dispositif optique vers une unité de stockage ou un moyen de traitement de ces données.
De préférence, ce dispositif optique est une caméra numérique. Cette dernière peut ainsi comporter une matrice de capteurs CCD.
De manière avantageuse, les signaux émis par le ou les capteurs du dispositif optique sont des signaux de communication sans fil. De préférence, il en est de même pour les signaux émis par le ou les capteurs du détecteur infrarouge.
Ces signaux de communication sans fil peuvent être basés sur les protocoles suivants : IEEE 802.11 b/g/n (Wi-Fi), IEEE 802.15.1 (Bluetooth) ou encore GSM ou GPRS.
Alternativement, ces signaux sont transmis via un réseau filaire (électrique, téléphonique, Ethernet) pour éviter de potentielles perturbations.
Le système comporte avantageusement un moyen de traitement de ces signaux comprenant une unité de calcul, un clavier, un écran d'affichage et une unité de stockage pour le stockage des ensembles de données correspondant à différents fers. Sur cette unité de calcul, sont exécutés un ou plusieurs logiciels de traitement de données pour traiter les signaux reçus du ou des capteurs du dispositif optique et du détecteur infrarouge et les stocker.
Dans différents modes de réalisation particuliers de ce système, chacun ayant ses avantages particuliers et susceptibles de nombreuses combinaisons techniques possibles:
- ce système comprend un élément d'entrée transparent à la lumière visible et dans une bande infrarouge, ledit élément d'entrée étant porté par un capot et destiné à recevoir en appui ledit, ou être placé à proximité dudit, sabot à ferrer, ledit capot portant ledit dispositif optique et ledit détecteur infrarouge sensible dans ladite bande infrarouge.
- ce système comporte un élément de renvoi réfléchissant le rayonnement incident dans ladite bande infrarouge vers ledit détecteur infrarouge, ledit élément de renvoi étant porté par ledit capot, ledit élément de renvoi étant transparent dans le visible.
Alternativement, ce système comporte un élément de renvoi réfléchissant le rayonnement incident dans le visible, ledit élément de renvoi étant porté par ledit capot, ledit élément de renvoi étant transparent dans ladite bande infrarouge.
A titre d'exemple, il peut s'agir d'un filtre d'interférence formé de plusieurs couches présentant une haute transparence pour la lumière visible et un haut pouvoir réfléchissant pour le rayonnement infrarouge dans ladite bande infrarouge.
De manière alternative, ce système peut comporter un système de déflexion optique et de déport d'images définissant une même ligne de visée pour ledit détecteur infrarouge et ledit dispositif optique. Cette ligne de visée est de préférence perpendiculaire ou sensiblement perpendiculaire audit élément d'entrée.
Le système comporte donc avantageusement une voie optique d'entrée commune au détecteur infrarouge et au dispositif optique.
- le dispositif optique est placé à distance de l'élément d'entrée tel qu'un hublot transparent, pour visualiser ledit sabot à ferrer de dessous.
L'axe optique du dispositif optique est par exemple perpendiculaire à l'élément d'entrée.
- le système comporte un dispositif optique sensible aux infrarouges et un filtre infrarouge pour ne laisser passer que les infrarouges au-delà d'une fréquence de coupure prédéterminée ou un programme d'ordinateur configuré pour traiter les images acquises par ledit dispositif optique sensible aux infrarouges et fournir des données uniquement dans les infrarouges.
L'axe optique de ce dispositif optique sensible à la lumière visible et aux infrarouges, peut alors être positionné de manière normal, ou sensiblement normal, au plan défini par l'élément d'entrée, transparent à la lumière visible et dans une bande infrarouge. L'obtention d'images dans l'infrarouge est alors soit obtenu en positionnant un filtre IR devant le dispositif optique, ce filtre IR laissant passer uniquement les infrarouges au-delà d'une fréquence de coupure prédéfinie, par exemple 720 nm, soit en traitant les données acquises par le dispositif optique au moyen d'un logiciel configuré pour donner des images infrarouges. Le système d'assistance comporte alors un unique dispositif optique, lequel sert également de détecteur infrarouge.
- ledit capot comporte un moyen d'affichage permettant d'afficher ledit sabot à ferrer tel que visualisé par ledit dispositif optique et/ou ledit détecteur infrarouge après conversion en signal vidéo dans un circuit de traitement électronique.
- le système comporte une découpeuse à commande numérique, un moyen d'impression ou un système pour la fabrication d'objets tridimensionnels, permettant de réaliser un patron d'un fer à cheval à partir de paramètres préalablement déterminés.
Ce système peut ainsi comprendre une imprimante numérique ou tridimensionnelle.
- ledit détecteur infrarouge est un détecteur infrarouge thermique. Ce détecteur comporte par exemple une matrice de capteurs tels que des microbolomètres ne nécessitant avantageusement pas de refroidissement.

A titre purement illustratif, de tels capteurs peuvent opérer dans la bande 8 à 14 µm.

De manière plus générale, la présente invention concerne aussi un procédé de fabrication d'un fer à cheval selon la revendication 1, dans lequel on réalise au moins les étapes suivantes:
a) visualiser au moins en partie le sabot à ferrer pour déterminer des mesures nécessaires à la réalisation d'un fer adapté à ce sabot,
b) traiter lesdites mesures pour en déduire une forme prévisionnelle du fer à cheval,
c) enregistrer des paramètres définitifs dudit fer à cheval, et
d) réalisation du fer à cheval à partir desdits paramètres définitifs.
Selon l'invention, à l'étape d), le fer à cheval est réalisé par un procédé de fabrication par impression tridimensionnelle.
L'impression tridimensionnelle (3D) peut être réalisée par impression 3D par dépôt de matière fondue, ou par impression 3D par liage de poudre, ou encore par photopolymérisation.
De préférence, une poudre métallique telle que de l'acier, du platine ou de l'aluminium, est utilisée comme matériau de base pour former le fer à cheval par impression tridimensionnelle.
De manière avantageuse, il est ainsi possible, après avoir déterminé les paramètres définitifs du fer à cheval, de fabriquer ce dernier directement. On obtient ainsi une réduction significative des délais de réalisation et la possibilité de ferrer le cheval sur place.
Un tel procédé d'impression tridimensionnelle qui permet de réaliser des pièces à géométries complexes, est également particulièrement adapté à la réalisation de fers orthopédiques lesquels peuvent présenter des géométries difficiles à réaliser par un maréchal-ferrant.
Le système d'assistance à la réalisation d'un fer à cheval, pour la mise en oeuvre du procédé tel que décrit précédemment comporte alors
- un dispositif optique,
- un détecteur infrarouge,
- ledit système étant agencé de sorte qu'au moins ledit dispositif optique visualise la partie dudit sabot destinée à recevoir ledit fer à cheval,
- des moyens assurant le transfert des données acquises par ledit détecteur infrarouge et ledit dispositif optique vers une unité de stockage ou un moyen de traitement de ces données, et
- une machine d'impression tridimensionnelle.
La machine d'impression tridimensionnelle est alors reliée au moyen de traitement des données tel qu'un ordinateur. Ce dernier comporte alors un programme d'ordinateur configuré pour contrôler cette machine d'impression afin de réaliser le fer à cheval à partir des paramètres définitifs de ce dernier.
Ce système d'assistance est avantageusement installé dans un engin qui peut soit être tracté soit être automoteur. Par exemple, il peut s'agir d'un véhicule motorisé de sorte que l'opérateur peut se rendre directement au plus près du lieu où se situe le cheval à ferrer.

### BREVE DESCRIPTION DES DESSINS

D'autres avantages, buts et caractéristiques particulières de la présente invention ressortiront de la description qui va suivre, faite, dans un but explicatif et nullement limitatif, en regard des dessins annexés, dans lesquels :
- la Figure 1 représente schématiquement une vue de côté d'un ensemble d'assistance à la détermination des paramètres d'un fer à cheval selon un mode de réalisation particulier de la présente invention, le pied d'un cheval étant placé en appui contre le hublot transparent placé à l'entrée de cet ensemble ;
- la Figure 2 est une vue de face d'une ferrure orthopédique obtenue par le procédé de l'invention selon un mode de réalisation particulier ;

### DESCRIPTION DETAILLEE DE MODE DE REALISATION DE L'INVENTION

Tout d'abord, on note que les figures ne sont pas à l'échelle.

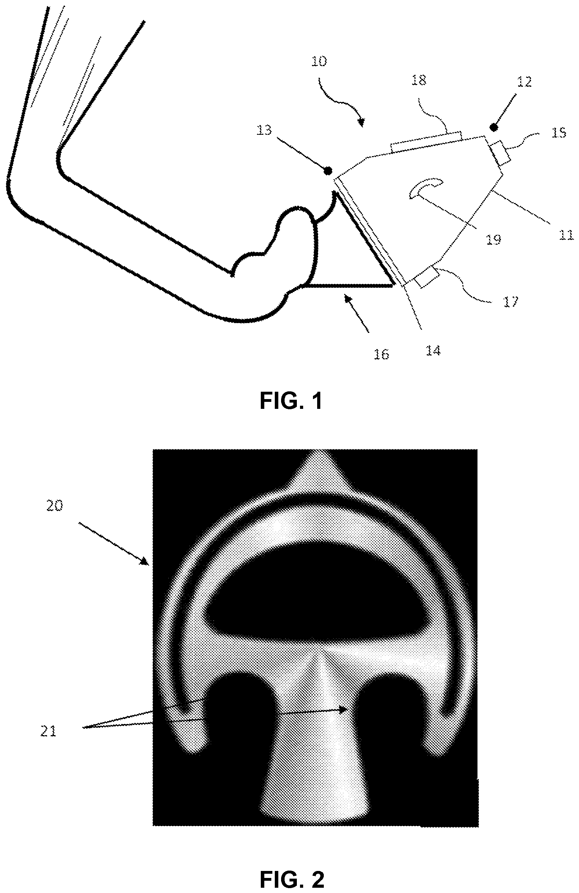

La Figure 1 montre de manière schématique une vue de côté d'un ensemble d'assistance à la détermination des paramètres d'un fer à cheval selon un mode de réalisation particulier de la présente invention.

Cet ensemble 10 comporte un capot 11 de forme allongée et de forme évasée en partant d'une extrémité arrière 12 vers son extrémité avant 13.

L'extrémité avant 13 de ce capot qui définit la voie d'entrée de cet ensemble 10, présente une forme cylindrique.

A l'entrée de cet ensemble 10, le capot 11 porte un hublot 14 transparent à la lumière visible et au rayonnement infrarouge dans une bande infrarouge, par exemple au rayonnement infrarouge jusqu'à 12 µm.

Avantageusement, ce hublot 14 transparent porte un quadrillage facilitant les mesures du sabot et, par conséquent, la détermination des paramètres du fer à cheval.

La partie arrière du capot 11 porte une caméra 15 numérique pour observer de dessous au travers du hublot 14, le pied 16 du cheval reçu en appui contre ce hublot 14 transparent. L'axe de visée de la caméra numérique est perpendiculaire ou sensiblement perpendiculaire à ce hublot 14. A titre purement illustratif, cette caméra numérique comporte une matrice de capteurs CCD.

L'invention prévoit ensuite le traitement des images obtenues, par un programme informatique exécuté sur une unité de calcul (non représentée) afin de pouvoir calculer les caractéristiques du sabot à ferrer et, par conséquent, déterminer à partir de ces caractéristiques, les mesures (courbure externe, taille et largeur de la surface portante (i.e. couverture), pinçon, étampures, contre-perçure, rolling, ...) du fer à cheval adapté pour ce sabot.

Sur cette unité de calcul, sont également stockées un ou plusieurs ensembles de données permettant, par exemple, une fois les mesures du fer à cheval déterminées, de proposer à l'utilisateur un ou plusieurs modèles de fer à cheval susceptibles de convenir pour le sabot à ferrer.

A titre d'exemple, ces modèles de fer à cheval varient en fonction de la perspective d'utilisation envisagée, et discipline, du cheval (loisir, trait, course, polo, ...), de sa race, de son âge, des conditions climatiques éventuelles (verglas, neige ...), des problèmes orthopédiques, etc.... afin de maintenir ou d'optimiser les performances locomotrices du cheval. La forme finale du fer à cheval est déterminée par le maréchal-ferrant et/ou le vétérinaire.

Le capot 11 de cet ensemble 10 porte également un détecteur 17 infrarouge thermique pour obtenir des images thermographiques du pied 16 du cheval.

Ces images traitées par un logiciel adapté exécuté sur l'unité de calcul permettent à l'utilisateur de détecter un ou plusieurs problèmes physiologiques (inflammation, lésion, réduction de la circulation sanguine, ...) dans le pied du cheval et de tenir compte de ce ou ces problèmes dans la définition finale (forme et/ou structure) du fer à cheval.
Eventuellement, l'entrée de cet ensemble 10 comporte également un filtre (non représenté) transparent uniquement dans la bande infrarouge auquel est sensible ce détecteur infrarouge 17. Ce filtre est mobile pour pouvoir être éloigné ou positionné en vis-à-vis de ce hublot 14 transparent.

Le capot 11 porte un élément de renvoi (non représenté) réfléchissant le rayonnement incident dans ladite bande infrarouge vers le détecteur infrarouge 17, cet élément de renvoi étant transparent dans le visible.

La caméra numérique 15 et le détecteur infrarouge 17 sont chacune reliées via un câble adapté (non représenté) à l'unité de calcul pour transmettre les signaux émis par leurs capteurs respectifs.

La surface externe du capot 11 reçoit également un écran 18 de visualisation permettant d'afficher en direct le sabot à ferrer tel qu'observé par la caméra numérique et/ou imagé par ledit détecteur infrarouge après conversion en signal vidéo dans un circuit de traitement électronique (non représenté).

Le capot 11 comporte avantageusement des anses 19 autorisant une manipulation aisée de l'ensemble 10 par l'utilisateur.

Le capot 11 peut encore comporter un orifice fileté destiné à coopérer avec un trépied (non représenté) pour assurer le support de l'ensemble 10 en position fixe.

La Figure 2 est une vue de face d'une ferrure orthopédique obtenue par le procédé de l'invention selon un mode de réalisation particulier.

## Revendications

1. Procédé de fabrication d'un fer à cheval, dans lequel on réalise les étapes suivantes :
a) visualiser au moins en partie le sabot (16) à ferrer pour déterminer des mesures nécessaires à la réalisation d'un fer adapté à ce sabot (16),
b) traiter lesdites mesures pour en déduire une forme prévisionnelle du fer à cheval,
c) enregistrement des paramètres définitifs dudit fer à cheval, et
d) réalisation du fer à cheval à partir desdits paramètres définitifs, **caractérisé en ce que**
- avant l'étape b), on détermine au moyen d'un détecteur (17) infrarouge l'existence éventuelle d'au moins une zone d'inflammation et/ou d'au moins une zone de réduction de la circulation sanguine du pied du cheval, dont le sabot (16) est destiné à recevoir ledit fer, et
- on tient compte à l'étape b), de l'existence d'au moins une telle zone pour déterminer ladite forme prévisionnelle et/ou ladite structure dudit fer à cheval.

2. Procédé selon la revendication 1, **caractérisé en ce que** l'étape de détection d'au moins une éventuelle zone d'inflammation et/ou d'au moins une zone de réduction de la circulation sanguine du pied du cheval est réalisée simultanément à la visualisation du sabot (16) à ferrer.

3. Procédé selon la revendication 1 ou 2, **caractérisé en ce qu'**à l'étape a), on acquiert au moins une image numérique d'au moins une partie du sabot (16) à ferrer.

4. Procédé selon l'une quelconque des revendications 1 à 3, **caractérisé en ce qu'**on réalise également avant l'étape b) au moins une image radiographique dudit pied au moyen d'une source X-ray portable et d'un imageur X-ray pour déterminer une zone éventuelle d'affection.

5. Procédé selon l'une quelconque des revendications 1 à 4, **caractérisé en ce qu'**avant l'étape c), on réalise les étapes suivantes : réaliser un patron à partir des paramètres dudit fer à cheval obtenus à l'étape b), placer ce patron sur le sabot (16) à ferrer et déterminer les paramètres définitifs dudit fer à cheval à fabriquer.

6. Procédé selon l'une quelconque des revendications 1 à 5, **caractérisé en ce que** l'étape b) comporte une étape de correction des paramètres du fer à cheval correspondant au moins à un ajustement de la courbe externe du fer à cheval.

7. Procédé selon l'une quelconque des revendications 1 à 6, **caractérisé en ce que** le pied du cheval présentant au moins une zone douloureuse ou une zone de réduction de la circulation sanguine, à l'étape b) on allège la structure du fer au droit de ladite au moins une zone de sorte à soulager celle-ci.

8. Procédé selon l'une quelconque des revendications 1 à 7, **caractérisé en ce qu'**à l'étape d), on réalise au moins une partie du fer à cheval par un procédé de fabrication par impression tridimensionnelle.

9. Procédé selon l'une quelconque des revendications 1 à 8, **caractérisé en ce qu'**on marque la surface externe dudit fer avec un identifiant unique dudit fer à cheval et éventuellement un identifiant du pied du cheval à ferrer auquel est destiné ledit fer à cheval.

10. Procédé selon l'une quelconque des revendications 1 à 9, **caractérisé en ce qu'**on réalise une finition de la couleur du fer à cheval ainsi obtenu par traitement d'au moins une partie de la surface externe dudit fer, ledit traitement comportant une étape d'anodisation et une étape de coloration, ou par dépose sur au moins une partie de la surface externe dudit fer d'un revêtement décoratif présentant une couleur déterminée.

11. Procédé selon la revendication 10, **caractérisé en ce que** déposant un revêtement décoratif résistant à l'usure, on choisit un métal précieux parmi le groupe comprenant l'Or, l'Argent, le Platine, le Palladium, le Rhodium, l'Irridium, l'Osmium, le Rhénium, le Ruthénium et/ou d'un alliage d'un de ces métaux avec un ou plusieurs autres métaux.

12. Système d'assistance à la réalisation d'un fer à cheval, pour la mise en oeuvre du procédé selon l'une quelconque des revendications 1 à 11, ledit système comprenant
- un dispositif (15) optique,
- un détecteur (17) infrarouge,
- ledit système étant agencé de sorte qu'au moins ledit dispositif (15) optique visualise la partie dudit sabot (16) destinée à recevoir ledit fer à cheval, et
- des moyens assurant le transfert des données acquises par ledit détecteur (17) infrarouge et ledit dispositif (15) optique vers une unité de stockage ou un moyen de traitement de ces données.

13. Système selon la revendication 12, **caractérisé en ce qu'**il comprend un élément d'entrée transparent à la lumière visible et dans une bande infrarouge porté par un capot, ledit élément d'entrée étant destiné à recevoir en appui ou être placé à proximité dudit sabot (16) à ferrer, ledit capot portant ledit dispositif (15) optique et ledit détecteur (17) infrarouge sensible dans ladite bande infrarouge.

14. Système selon la revendication 12 ou 13, **caractérisé en ce qu'**il comporte un dispositif optique sensible aux infrarouges et un filtre infrarouge pour ne laisser passer que les infrarouges au-delà d'une fréquence de coupure prédéterminée ou un programme d'ordinateur configuré pour traiter les images acquises par ledit dispositif optique sensible aux infrarouges et fournir des données uniquement dans les infrarouges.

15. Système selon la revendication 13 ou 14, **caractérisé en ce que** ledit capot comporte un moyen (18) d'affichage permettant d'afficher ledit sabot (16) à ferrer tel que visualisé par ledit dispositif (15) optique et/ou ledit détecteur (17) infrarouge après conversion en signal vidéo dans un circuit de traitement électronique.

16. Système selon l'une quelconque des revendications 12 à 15, **caractérisé en ce qu'**il comporte une découpeuse à commande numérique, un moyen d'impression ou un système pour la fabrication d'objets tridimensionnels, permettant de réaliser un patron d'un fer à cheval à partir de paramètres préalablement déterminés.

## Patentansprüche

1. Verfahren zum Herstellen eines Hufeisens, in welchem die folgenden Schritte durchgeführt werden:
a) wenigstens teilweises Visualisieren des zu beschlagenden Hufs (16), um die notwendigen Abmessungen für die Herstellung eines Hufeinens zu bestimmen, welches auf diesen Huf (16) angepasst ist,
b) Verarbeiten dieser Abmessungen, um eine vorläufige Form des Eisens abzuleiten,
c) Aufnehmen von endgültigen Parametern des Hufeisens, und
d) Herstellen des Hufeisens ausgehend von den endgültigen Parametern,
**dadurch gekennzeichnet, dass**
- vor Schritt b) mittels eines Infrarotdetektors (17) das mögliche Vorliegen von wenigstens einer Entzündungszone und/oder wenigstens einer Zone mit reduziertem Blutfluss am Bein des Pferds bestimmt wird, dessen Huf (16) dazu vorgesehen ist, das Eisen zu erhalten, und
- in Schritt b) das Vorliegen von wenigstens einer derartigen Zone berücksichtigt wird, um die vorläufige Form und/oder die Struktur des Hufeisens zu bestimmen.

2. Verfahren nach Anspruch 1, **dadurch gekennzeichnet, dass** der Schritt des Detektierens von wenigstens einer möglichen Entzündungszone und/oder wenigstens einer Zone mit reduziertem Blutfluss am Bein des Pferds gleichzeitig mit dem Visualisieren des zu beschlagenden Hufs (16) durchgeführt wird.

3. Verfahren nach Anspruch 1 oder 2, **dadurch gekennzeichnet, dass** bei Schritt a) wenigstens ein digitales Bild von wenigstens einem Teil des zu beschlagenden Hufs (16) aufgenommen wird.

4. Verfahren nach einem der Ansprüche 1 bis 3, **dadurch gekennzeichnet, dass** ebenfalls vor dem Schritt b) wenigstens ein Röntgenbild des Beins mittels einer tragbaren Röntgenquelle und eines Röntgen-Bildaufnehmers aufgenommen wird, um eine mögliche Erkrankungszone zu bestimmen.

5. Verfahren nach einem der Ansprüche 1 bis 4, **dadurch gekennzeichnet, dass** vor dem Schritt c) die folgenden Schritte durchgeführt werden: Erstellen einer Vorlage ausgehend von den Parametern des Hufeisens, die in Schritt b) erhalten werden, Platzieren dieser Vorlage an dem zu beschlagenden Huf (16) und Bestimmen der endgültigen Parameter des herzustellenden Hufeisens.

6. Verfahren nach einem der Ansprüche 1 bis 5, **dadurch gekennzeichnet, dass** der Schritt b) einen Korrekturschritt der Parameter des Hufeisens umfasst, welche wenigstens einer Anpassung der Außenkrümmung des Hufeisens entsprechen.

7. Verfahren nach einem der Ansprüche 1 bis 6, **dadurch gekennzeichnet, dass** das Bein des Pferds wenigstens eine schmerzhafte Zone oder eine Zone mit reduziertem Blutfluss aufweist, wobei in Schritt b) die Struktur des Eisens dieser wenigstens einen Zone verringert wird, um selbige zu entlasten.

8. Verfahren nach einem der Ansprüche 1 bis 7, **dadurch gekennzeichnet, dass** in Schritt d) wenigstens ein Teil des Hufeisens durch einen dreidimensionalen Druck-Herstellungsvorgang hergestellt wird.

9. Verfahren nach einem der Ansprüche 1 bis 8, **dadurch gekennzeichnet, dass** die Außenfläche des Eisens mit einer eindeutigen Kennung des Hufeisens markiert wird und gegebenenfalls einer Kennung des zu beschlagenden Beins des Pferds, für welches das Hufeisen vorgesehen ist.

10. Verfahren nach einem der Ansprüche 1 bis 9, **dadurch gekennzeichnet, dass** eine Endbearbeitung der Farbe des derart erhaltenen Hufeisens mittels einer Behandlung von wenigstens einem Teil der Außenfläche des Eisens durchgeführt wird, wobei die Behandlung einen Schritt einer Anodisierung und einen Schritt einer Färbung umfasst, oder indem an wenigstens einem Teil der Außenfläche des Eisens eine dekorative Beschichtung aufgebracht wird, welche eine vorbestimmte Farbe aufweist.

11. Verfahren nach Anspruch 10, **dadurch gekennzeichnet, dass** beim Aufbringen einer widerstandsfähigen dekorativen Beschichtung ein Edelmetall aus der Gruppe ausgewählt wird, welche Gold, Silber, Platin, Palladium, Rhodium, Iridium, Osmium, Rhenium, Ruthenium und/oder eine Legierung von einem dieser Metalle mit einem oder mehreren anderen Metallen umfasst.

12. System zur Unterstützung der Herstellung eines Hufeisens zum Ausführen des Verfahrens nach einem der Ansprüche 1 bis 11, wobei das System umfasst:
- eine optische Vorrichtung (15),
- einen Infrarotdetektor (17),
- wobei das System derart eingerichtet ist, dass wenigstens die optische Vorrichtung (15) den Teil des Hufs (16) visualisiert, welcher dazu vorgesehen ist, das Hufeisen zu erhalten, und
- Mittel, welche die Übertragung der durch den Infrarotdetektor (17) und die optische Vorrichtung (15) aufgenommenen Daten zu einer Speichereinheit oder einem Mittel zur Verarbeitung dieser Daten sicherstellen.

13. System nach Anspruch 12, **dadurch gekennzeichnet, dass** es ein transparentes Eingangselement für sichtbares Licht und Licht in einem Infrarotband umfasst, welches von einer Haube getragen ist, wobei das Eingangselement dazu vorgesehen ist, den zu beschlagenden Huf (16) stützend aufzunehmen oder in seiner Nähe angeordnet zu werden, wobei die Haube die optische Vorrichtung (15) und den in dem Infrarotband empfindlichen Infrarotdetektor (17) trägt.

14. System nach Anspruch 12 oder 13, **dadurch gekennzeichnet, dass** es eine im Infraroten empfindliche optische Vorrichtung und einen Infrarotfilter umfasst, um lediglich das Infrarote jenseits einer vorbestimmten Abschneidefrequenz durchzulassen, oder ein Computerprogramm, welches dazu eingerichtet ist, die von der im Infraroten empfindlichen optischen Vorrichtung aufgenommenen Bilder zu verarbeiten und Daten lediglich im Infraroten zu liefern.

15. System nach Anspruch 13 oder 14, **dadurch gekennzeichnet, dass** die Haube ein Anzeigemittel (18) umfasst, welches es erlaubt, den zu beschlagenden Huf (16) so anzuzeigen, wie er von der optischen Vorrichtung (15) und/oder dem Infrarotdetektor (17) visualisiert wird, nach einer Umwandlung des Videosignals in einer elektronischen Verarbeitungsschaltung.

16. System nach einem der Ansprüche 12 bis 15, **dadurch gekennzeichnet, dass** es eine digital gesteuerte Schneidevorrichtung, ein Mittel zum Drucken oder ein System zur Herstellung von dreidimensionalen Objekten umfasst, welche/s es erlaubt, eine Vorlage eines Hufeisens ausgehend von zuvor bestimmten Parametern herzustellen.

## Claims

1. Method for manufacturing a horseshoe, wherein the following steps are carried out:
a) at least partially visualizing the hoof (16) to be shod in order to determine the measurements necessary to produce a shoe adapted to this hoof (16),
b) processing said measurements in order to deduce therefrom a provisional form of the horseshoe,
c) recording the definitive parameters for said horseshoe, and
d) producing the horseshoe from said definitive parameters, **characterized in that**
- before step b), the potential existence of at least one area of inflammation and/or at least one area of reduced blood circulation of the horse's foot, the hoof of which is intended to receive said shoe, is determined by means of an infrared detector (17), and
- the existence of at least one such zone is taken into account in step b) in order to determine said provisional form and/or said structure of said horseshoe.

2. Method according to Claim 1, **characterized in that** the step of detecting at least one potential area of inflammation and/or at least one area of reduced blood circulation of the horse's foot is carried out simultaneously to the visualization of the hoof (16) to be shod.

3. Method according to Claim 1 or 2, **characterized in that**, in step a), at least one digital image is acquired of at least one part of the hoof (16) to be shod.

4. Method according to any one of Claims 1 to 3, **characterized in that**, also before step b), at least one radiographic image of said foot is taken by means of a portable X-ray source and an X-ray imager in order to determine a potential afflicted area.

5. Method according to any one of Claims 1 to 4, **characterized in that**, before step c), the following steps are carried out: creating a template from the parameters for said horseshoe obtained in step b), placing this template on the hoof (16) to be shod, and determining the definitive parameters for said horseshoe to be manufactured.

6. Method according to any one of Claims 1 to 5, **characterized in that** step b) comprises a step of correction of the parameters for the horseshoe, corresponding to at least one adjustment of the external curve of the horseshoe.

7. Method according to any one of Claims 1 to 6, **characterized in that**, with the foot of the horse having at least one painful area or an area of reduced blood circulation, the structure of the shoe is lightened in step b) in line with said at least one area, so as to bring relief thereto.

8. Method according to any one of Claims 1 to 7, **characterized in that**, in step d), at least part of the horseshoe is produced by a three-dimensional printing manufacturing method.

9. Method according to any one of Claims 1 to 8, **characterized in that** the external surface of said shoe is marked with a unique identifier for said horseshoe and optionally an identifier of the foot of the horse to be shod for which said horseshoe is intended.

10. Method according to any one of Claims 1 to 9, **characterized in that** a colour finish is produced on the horseshoe thus obtained, by treating at least a part of the external surface of said shoe, said treatment comprising a step of anodizing and a step of colouring, or by depositing a decorative coating having a determined colour on at least a part of the external surface of said shoe.

11. Method according to Claim 10, **characterized in that**, when depositing a wear-resistant decorative coating, a precious metal from the group comprising gold, silver, platinum, palladium, rhodium, iridium, osmium, rhenium, ruthenium and/or an alloy of one of these metals with one or more other metals, is chosen.

12. System for aiding the production of a horseshoe, for carrying out the process according to any one of Claims 1 to 11, said system comprising:
- an optical device (15),
- an infrared detector (17),
- said system being arranged such that at least said optical device (15) visualizes the part of said hoof (16) intended to receive said horseshoe, and
- means for transferring the data acquired by said infrared detector (17) and said optical device (15) to a storage unit or a means for processing this data.

13. System according to Claim 12, **characterized in that** it comprises an input element that is transparent to light that is visible and in an infrared band, borne by a cover, said input element being intended to receive in abutment or be placed in proximity to said hoof (16) to be shod, said cover bearing said optical device (15) and said infrared detector (17) that is sensitive in said infrared band.

14. System according to Claim 12 or 13, **characterized in that** it comprises an infrared-sensitive optical device and an infrared filter so as to only allow passage of infrared radiation beyond a predetermined cut-off frequency, or a computer program configured to process the images acquired by said infrared-sensitive optical device and to provide data only in the infrared range.

15. System according to Claim 13 or 14, **characterized in that** said cover comprises a display means (18) making it possible to display said hoof (16) to be shod as visualized by said optical device (15) and/or said infrared detector (17) after conversion to video signal in an electronic processing circuit.

16. System according to any one of Claims 12 to 15, **characterized in that** it comprises a digitallycontrolled cutting machine, a means for printing or a system for manufacturing three-dimensional objects, making it possible to produce a horseshoe template from predetermined parameters.
